(19)

Europäisches Patentamt

European Patent Office

Office européen des brevets

(11) EP 4 342 538 A1

(12) EUROPEAN PATENT APPLICATION

(43) Date of publication:
27.03.2024 Bulletin 2024/13

(21) Application number: 22196655.9

(22) Date of filing: 20.09.2022

(51) International Patent Classification (IPC):
A61P 31/04 (2006.01)        C12N 7/00 (2006.01)
C12P 21/00 (2006.01)

(52) Cooperative Patent Classification (CPC):
A61P 31/04; C07K 14/005; C12N 7/00;
C12N 2795/10221; C12N 2795/10222;
C12N 2795/10232; C12N 2795/10251

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicants:
• Technische Universität München
80333 München (DE)
• Falgenhauer, Elisabeth
81687 Munich (DE)

(72) Inventors:
• SIMMEL, Friedrich
80336 München (DE)
• VOGELE, Kilian
82399 Raisting (DE)
• VON SCHÖNBERG, Sophie
85386 Eching (DE)

(74) Representative: Weickmann & Weickmann
PartmbB
Postfach 860 820
81635 München (DE)

(54) **SIMULTANEOUS PRODUCTION OF STRUCTURAL PROTEINS FROM HETEROLOGOUS BACTERIOPHAGE IN CELL-FREE EXPRESSION SYSTEM**

(57)     The present invention relates to multi-peptide structures comprising at least one heterogenous functional site wherein the at least one heterogenous functional site is composed of at least two homologous peptides, which differ by at least one amino acid, a method for providing such multi-peptide structures, compositions comprising such multi-peptide structures as well as the use of such multi-peptide structures and compositions as an universal anti-microbial agent, in particular in medicine, chemistry, biotechnology, agriculture and/or food industry.

FIGURE 2

**Description**

The present invention relates to multi-peptide structures comprising at least one heterogenous functional site wherein the at least one heterogenous functional site is composed of at least two homologous peptides, which differ by at least one amino acid, a method for providing such multi-peptide structures, compositions comprising such multi-peptide structures as well as the use of such multi-peptide structures and compositions as an universal anti-microbial agent, in particular in medicine, chemistry, biotechnology, agriculture and/or food industry.

Bacteriophages (herein also called "phages") are viruses that specifically infect a host bacterium and proliferate at the expense of this host. They are composed of proteins that encapsulate a DNA or RNA molecule. Within their lytic state, Bacteriophages replicate within a host bacterium by injecting their viral genetic material into the host cell effectively taking over the cells functions for the production of progeny bacteriophage which leads to the rupture of the cell wall and subsequent bacterial cell death.

The biotechnological fields of application of bacteriophages are very broad and extend from evolution-based selection methods, like the evolutionary improvement of the activity of enzymes and other proteins, to phage display by which biological drug substances, e.g. therapeutic antibodies, could be generated and optimized, as well as the application of the bacteriophages themselves as a substitute for antibiotics.

The later use is based on the natural characteristics of bacteriophages to specifically affect bacteria, in particular pathogenic bacteria, and kill them, usually by lysing the bacteria or by inhibiting the replication system of the bacteria.

This approach that has been applied for a longer time to fight microbial infections and gets more and more attention as the number and spread of multi-drug resistant bacteria strains increases strongly worldwide. Methicillin-resistant Staphylococcus aureus (MRSA) bacteria, for example, is an increasingly common form of infection, often acquired through transmission in hospitals. MRSA infections are extremely difficult to treat using conventional antibiotics. The development of novel antibiotics is significantly slower than this development.

The development of phage-based therapeutics and diagnostics is, however, prevented by problems in the production and/or modification of bacteriophages. The complexity of the modification of bacteriophages is mainly due to the difficulty of changing the genome of bacteriophages.

These two main aspects of the production of bacteriophages, i.e. complexity of modification as well as simple and safe provision, have both been addressed at the same time by the methods and multi-peptide structures, i.e. synthetic bacteriophages, according to the present invention.

A first aspect of the present invention relates to a multi-peptide structure comprising at least one heterogenous functional site wherein the at least one heterogenous functional site is composed of at least two homologous peptides, which differ by at least one amino acid.

As understood herein, a "homogenous functional site" is composed of peptides, in particular non-engineered peptides of the same type of bacteriophage, whereas a "heterogenous functional site" is composed of homologous peptides differing by at least one amino acid, preferably derived from different bacteriophages.

The terms "peptide" and "protein" are understood by the person skilled in the art and may be used interchangeable where appropriate.

The term "homologous" as used herein is understood by the person skilled in the art and relates to proteins which are similar in position and structure and possibly evolutionary origin but not necessarily function. For example, the at least two homologous proteins comprised by the inventive multi-peptide structure show preferably a similar or almost identical structure. At least the structural elements necessary for assembly and/or interaction with other multi-peptide structure peptides are similar, preferably identical. Within a bacteriophage structure, the two homologous proteins are arranged in the same functional site as herein described. However, the at least two homologous proteins may differ in function such as receptor/host binding and/or specificity.

The inventive multi-peptide structure comprises at least one functional site wherein the at least one functional site being composed of at least two homologous peptides.

A "functional site" as herein understood denotes a component and/or a spatial position of peptides in the multi-peptide structure which fulfills a given function.

Generally, such functional sites as herein understood may be set up by single monomeric peptides or may be composed of a couple proteins, which may be identical or not, which assemble and/or bind together to form a structure like a capsid, tail or tail fibre. Thus, functional sites may be composed by monomeric peptides, oligomeric peptides as well as several different proteins. For example, capsid structures may be set up of several identical monomeric peptides, T7 tail fibres are set up as a homotrimer of three identical peptides and the T4 long fibre tail is set up of different proteins.

The multi-peptide structure can comprise several different functional sites. In general, a "functional site" according to the present invention may be selected, for example, from head (capsid), tail, spike, sheath, tube, baseplate and tail fibre components of a bacteriophage. Capsid and tail fibre functional sites are in particular preferred.

The inventive multi-peptide structure can contain a given functional site, i.e. a type of functional site, several times, e.g. one to six times, such as a fibre tail.

For example, if the functional site comprising homologous proteins is a bacteriophage head or capsid component, there is one corresponding functional site in the multi-peptide structure; if the functional site comprising homologous proteins is a tail fibre there are usually one to six, preferably six, corresponding functional sites in the multi-peptide structure. For example, according to one embodiment, the inventive multi-peptide structure may comprise a given type of functional site only one time, e.g. like a capsid, which means that the at least two homologous proteins are both present within this functional site.

According to one embodiment, the multi-peptide structure may comprise a given type of functional site at least two times, e.g. like a fibre tail. In such an embodiment the at least two homologous peptides may be present at the same functional site position, if such functional site is set up by more than one protein, and/or may be present at the different same type functional site positions, in particular if the functional site is set up by one protein only.

"Homologous proteins" as used in the context herein refers to proteins which are not identical, i.e. which differ by at least one amino acid, i.e. do not have the same amino acid sequence" but fulfil essentially the same functional and/or structural role, e.g. have essentially the same specificity to other bacteriophage proteins to be assembled with, preferably self-assembled, in the inventive multi-peptide structure as defined above. Homologous peptides may show 80% or more sequence identity, preferably 85% or more, 90% or more or even 95% or more. Homologous peptides may differ by 1-30, or 1-25 or 1-20 or 1-15, or 1-10 or 1-5 amino acids. "Sequence identity" as herein understood refers to the occurrence of exactly the same amino acid in the same position in aligned sequences over the entire length. The homologous proteins can show conservative amino acid substitutions.

Their specificity of homologous proteins for targets, such as cell membranes or cell receptors, can be different.

According to a preferred embodiment the at least two homologous bacteriophage peptides are selected from the group comprising tail fibre proteins or tail fibre loops and/or capsid proteins, in particular receptor binding proteins. A tail fibre may be composed of homologous proteins including receptor binding proteins. For example, the long tail fibre of E. *coli* phage T4 is composed of four different proteins, wherein the distal subunit of the fibre, gp37, mediates receptor binding with its carboxy-terminal region.

The at least two homologous peptides, preferably derived from different bacteriophages, may be part of the same functional site in the multi-peptide structure, preferably a synthetic bacteriophage, which means that these two homologous bacteriophage peptides are both, for example, tail fibre proteins, tail fibre loops, capsid proteins, receptor binding proteins, etc.

According to a preferred embodiment, the multi-peptide structure resembles a bacteriophage structure; in other words, a multi-peptide structure according to the invention is preferably a "synthetic bacteriophage" or "engineered bacteriophage".

In general, the basic structure of bacteriophages consists of a core of nucleus material, i.e. a nucleic acid, preferably genome, surrounded by a protein capsid (head).

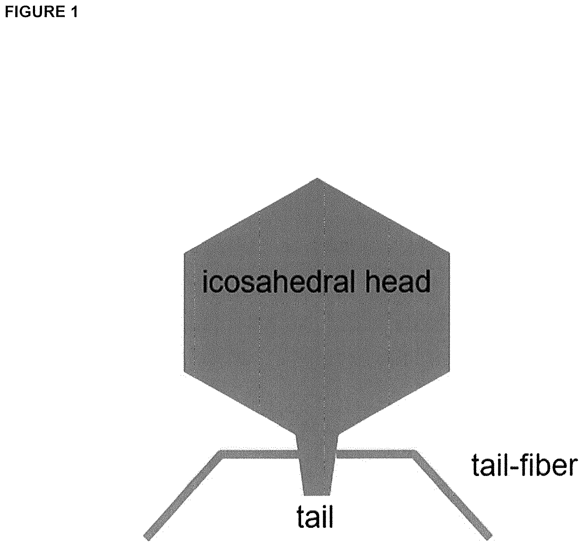

Bacteriophages may exist in three basic structural forms, an icosahedral-like head with a tail, an icosahedral-like head without a tail and a filamentous form. Basic structures comprising an icosahedral head with a tail are preferred. Such a structure is shown in FIG. 1, for instance.

However, according to another preferred embodiment, the head may be deleted and/or the remaining bacteria penetration structure be altered. As understood herein such a "headless bacteriophage" is also comprised by the term synthetic bacteriophage. In particular, such "headless bacteriophages" can induce cell death without replication of the bacteriophage.

Despite the enormous differences in the shape of viruses, generalized principles for the structure of the protein coat can be found, since it is based on the self-assembly of smaller asymmetric building blocks.

For example, the general structure of most capsids is a polyhedron. This capsid can be constructed by applying symmetry principles with a small number of genes.

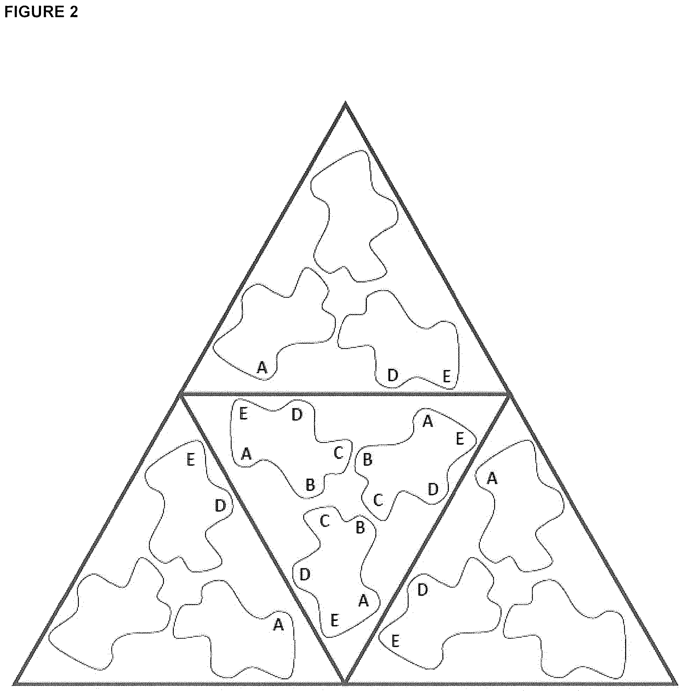

In order to arrange the asymmetric building blocks to form a symmetric structure, three of the building blocks must be placed in a triangular shape to form an icosahedron. In such a triangle, the building blocks have six equivalent contact points AE, three more equivalent contact points BC, and three similar contact points DD, as shown in Figure 2.

Since the same contact points are used multiple times, a symmetric structure can be built from these triangular lattices. An icosahedron can be constructed from 20 equilateral triangles with 12 vertices. Since a triangle consists of 3 building blocks, the smallest possible icosahedron consists of 60 subunits. To increase the size of the icosahedral capsid, new smaller triangles must be built into the original triangles of the icosahedron. Since the structure must be symmetrical, only a certain number of new triangles can be added to the icosahedral structure. These newly added triangles form hexamers, while pentamers are formed at the vertices.

The number of newly formed hexamers H can be calculated using the triangulation number T, which indicates how many times the original triangle of the icosahedron is divided. The formula for this is $H = 10 \cdot (T-1)$. The triangulation number T can be calculated using two variables $h \geq 1$ and $k \geq 0$, which must be integers. h is the number of steps in a straight line from one pentamer to the nearest point of the adjacent pentamer, while k is the number of steps required in a straight line from the point taken with h steps to the adjacent pentamer, at a 60° angle from it. Thus, the triangulation number T itself is calculated via:

$$T=h^2+h\cdot k+k^2$$

There are several examples of such icosahedral structures in viruses, such as rhinovirus and the virus family Parvoviridae with T=1, hepatitis B virus with T=3, and papillomaviruses and polyomaviruses with T=7 as well as with regard to the head of bacteriophages Escherichia-phage T7 with T=7, Enterobakteriophage MS2 with T=3 or Escherichia-phage T5 with T=13.

Considering such symmetry rules and considerations, capsid proteins can self-assemble into a capsid structure. Similar considerations apply to the multi-peptide structure of the present invention as well as other components of synthetic bacteriophages such as fiber tails and the tail itself.

According to a preferred embodiment, the multi-peptide structure comprises at least two homologous tail fibre proteins, preferably derived from different bacteriophages and/or at least two homologous capsid proteins, preferably derived from different bacteriophages.

According to a preferred embodiment the multi-peptide structure is self-assembled. "Self-assembled" within the present invention refers to a multi-peptide structure, in particular synthetic bacteriophage, that has been spontaneously formed to a structural organization or pattern by the specific interaction of involved proteins. Such structural organization may be a synthetic bacteriophage comprising all components of a wild-type bacteriophage, or a synthetic bacteriophage not comprising all components of a wild-type bacteriophage such as a headless bacteriophage. The proteins providing a multi-peptide structure as herein described self-assemble via specific non-covalent interaction, i.e. the proteins providing a self-assembled multi-peptide structure are non-covalently bonded to each other.

The inventive multi-peptide structure is preferably capable of binding on the surface of a cell, in particular a bacterial cell. The homologous tail fibre proteins may be specific for the same or different hosts, wherein being specific for different hosts is preferred. The inventive multi-peptide structure or synthetic bacteriophage may be called multi-functional. "Host" as used herein describes the whole breadth of organisms comprising single species as well as strains which can be effected by the inventive multi-peptide structure or synthetic bacteriophage.

For example, homologous tail fibre proteins may be specific for gram positive and/or gram negative bacteria, in particular gram positive and gram negative bacteria. Having homologous tail fibre proteins and/or receptor binding proteins incorporated the host range of the inventive multi-peptide, in particular synthetic bacteriophage range, may be enhanced.

The homologous peptides may be derived from different bacteriophages, in particular different wild-type bacteriophages. However, they may be also derived from the same bacteriophage, wherein at least one peptide has been engineered. According to another preferred embodiment all different homologous peptides are engineered. According to yet another embodiment, the bacteriophages are derived from the same wild-type but engineered in a different way.

The different bacteriophages where the homologous peptides are derived from, in particular wild-type bacteriophages, may be selected from the group comprising Caudovirales (familiy of Myoviridae, Autographiviridae, Podoviridae, Ackermannviridae, and Siphoviridae), Leviviridae, Tectiviridae, Corticoviridae, Plasmaviridae, Sphaerolipoviridae, Inoviridae, Microviridae, Picobirnaviridae and/or Cystoviridae.

According to a preferred embodiment, the inventive multi-peptide structure comprises a bacteriophage derived nucleic acid, in particular bacteriophage genome, encoding at least one homologous peptide less than comprised by the inventive multi-peptide structure; i.e. after replication, the 1. generation multi-peptide structure, in particular synthetic bacteriophage, differs from the parent multi-peptide structure as the 1. generation multi-peptide structure comprises at least one homologous peptide, being not encoded, less than the claimed multi-peptide structure.

Such bacteriophage derived nucleic acid may be any genetic material or nuclear material of bacteriophages, in particular bacteriophage a genome, which can be either DNA or RNA and/or variants thereof, which can either be double-stranded or single-stranded. Such genetic material or nuclear material of bacteriophages, in particular of a bacteriophage genome, may also be engineered. However, according another embodiment such bacteriophage derived nucleic acid is a unmodified bacteriophage genome, in particular wild-type genome. According to certain embodiments, the genetic material facilitates the replication of the multi-peptide structure, in particular of the synthetic bacteriophage.

Engineering, e.g. peptide engineering, may expand the abilities of the underlying protein by approaches known to the person skilled in the art und includes in particular site-specific mutations such as insertions, deletions and/or point mutations of the corresponding protein encoding nucleic acids, incorporation of non-natural amino acids as well as the addition of further functional groups such as labeling groups, active agents etc. As understood by the person skilled in the art such engineering can be performed on the nucleic acid level as well as on the protein level.

Due to the degeneracy of the genetic code, any nucleic acid sequence can be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. Such a conservative change may be, for example, done for improved expression of the protein in a given expression system.

According to a preferred embodiment, at least one of the receptor binding proteins and/or capsid forming proteins comprises non-naturally generated mutations, e.g. resulting from deletions, insertions and/or substitutions within the corresponding encoding nucleic acid as described above.

According to a preferred embodiment the multi-peptide structure, in particular synthetic bacteriophage, may be labelled. Such a label may be, for example, a marker, or an active agent. A "marker" as understood herein may be a tag such as biotin, a his-tag or any other tag recognizable by a binding partner such as an antibody, useable, for example, to isolate the bacteriophage and/or tags masking bacteriophages from the immune system of an object to be treated, such as peptides of human cells to mask the bacteriophage from the human immune system. Markers suitable for use in affinity purification processes include glutathione-5-transferase (GST), protein A, ScFv and lectins. A marker may be also a dye or a fluorescent label, e.g. for use in detection assays. Other modifications of the bacteriophage may be made, e.g. for reducing bacteriophage antigenicity, including use of a PEG (polyethyleneglycol) conjugate or a polysialic acid conjugate. An "active agent" as understood herein enhance the lethality of the bacteriophage to the bacterial host. The label may be incorporated at the DNA level, may be attached chemically at the phage surface or may even self-assemble into the inventive multi-peptide structure, in particular synthetic bacteriophage, within the inventive method described herein. Such a label, marker and/or tag may be added to any peptide of the inventive multi-peptide structure.

Another aspect of the invention relates to a method, in particular *in vitro* method, for providing multi-peptide structures, in particular synthetic bacteriophages, comprising the steps of

(a) providing an expression system, in particular a cell-free expression system,
(b) adding nucleic acids encoding homologous proteins, preferably of at least two different bacteriophages, in particular homologous phage tail fibre proteins and/or homologous phage capsid proteins, to the expression system,
(c) expressing the nucleic acids encoding the homologous proteins, preferably of the at least two different bacteriophages, in particular homologous phage tail fibre proteins and/or homologous phage capsid proteins, preferably of different bacteriophages,
(d) assembling of the expressed homologous proteins, preferably of the at least two bacteriophages, in particular homologous phage tail fibre proteins and/or homologous phage capsid proteins, preferably of different bacteriophages, to provide the assembled multi-peptide structures, in particular the synthetic bacteriophages, and optional
(e) isolating of the assembled multi-peptide structures, in particular the synthetic bacteriophages.

According to an especially preferred embodiment, the inventive method is an "all *in vitro* method". According to the present invention, this means that neither for replication of nucleic acids, in particular synthetic bacteriophages, nor the expression of such nucleic acids as well as the assembly of the corresponding proteins any cell, virus or bacteriophage based system is necessary.

The nucleic acids encoding proteins of at least two different viruses according to step (b) may be added in a predetermined ratio to determine the composition of the assembled multi-protein structure, in particular synthetic bacteriophage, to be provided. Such nucleic acids may be DNA and/or RNA molecules, such as PCR products, plasmid vectors, native DNA and/or RNA molecules, chemically or biologically synthesized molecules, artificial chromosomes, in particular yeast artificial chromosomes and/or bacterial artificial chromosomes, etc. According to a preferred embodiment, the DNA and/or RNA molecules are essentially complete genomes of different bacteriophages.

The at least two different bacteriophages referred to in step (b) may be at least two different wild-type bacteriophages, a first wild-type bacteriophage and at least one mutated or engineered bacteriophage derived from said first wild-type bacteriophage or at least two mutated or engineered bacteriophages derived from the same wild-type bacteriophage. By varying the concentration of the added coding genetic information, it is possible to adjust the final concentration of the resulting multi-peptide structure for further use.

At least one of the protein encoding nucleic acids may be modified, in particular modified by non-naturally mutations such as deletion, insertion and/ or substitution as described herein above.

At least one of the homologous proteins is preferably a capsid protein and/or tail fibre protein.

The cell-free expression system is a preferably host independent system. The term "cell free" is understood by the person skilled in the art and refers to "substantially free of".

A "cell-free expression system" as understood herein comprises a complete transcription and translation machinery for transcription and expression of a virus or bacteriophage nucleic acid, in particular a bacteriophage genome.

A preferred example for a cell free expression system is a cell lysate. Using such a cell lysate it is possible to synthesize several proteins or metabolites at the same time.

"Cell lysate" according to the present invention refers to a fluid comprising the components of cells from which it is derived after lysis. Lysis methods are known to the person skilled in the art and break down the membrane of a cell, for example, by viral, enzymatic, or osmotic mechanisms that compromise its integrity. Such cell lysate is essentially void of intact cells, i.e. cell-free. The terms "cell lysate" and "cell extract" can be used herein interchangeably.

After purification this lysate is essentially free, preferably free, of host DNA and makes an expression of the desired protein possible by the external addition of DNA, in particular a amplified bacteriophage genome as herein described. Thus, the used cell lysate is preferably free of nucleic acids, in particular DNA, and/or membranes from the cells of which it is derived. In a preferred embodiment, using standard techniques no host DNA can be detected in the purified lysate.

Cell lysates used according to the present invention may be derived from microorganisms, in particular bacteria such as pathogenic bacteria, E. *coli,* yeast, insects, mammals and/or plants, in particular such as wheat or rice, or may be even artificial. "Microorganism" refers to a bacterium or an archaeon. Preferably, the microorganism is a bacterium.

A number of cell-free expression systems is known to a person skilled in the art und may be applied according to the present invention. For example, the "PURE" system consists of several isolated proteins (Shimizu et al.) while untreated cell lysates (crude extracts) such as of E. *coli* include almost all intracellular proteins, even those that are not necessary for expression.

The use of E. *coli* lysate, in particular crude E. *coli* lysate, is a further preferred embodiment. A preferred example of crude E. *coli* lysate is E. *coli* S30 cell extract produced by a method based on the protocol described in E. Falgenhauer et al. (Falgenhauer, S. von Schönberg, C. Meng, A. Muckl, K. Vogele, Q. Emslander, C. Ludwig, F. C. Simmel, ChemBioChem 2021, 22, 2805).

External factors such as energy carriers, co-factors, amino acids, polymerases, transcription regulatory factors, chaperons, DNA stabilization agents (e.g. GamS and/or Chi6 DNA) may be added to enhance or otherwise improve the reaction. Such factors may be added as proteins and/or as nucleic acid encoding a respective protein.

According to a preferred embodiment, polyethylene glycol (PEG) may be added to the cell extract to increase molecular crowding. PEG binds water, creating a more concentrated environment. This may be advantageous because there is up to 25 to 30 times less protein concentration in the cell extract than in the cytosol of a bacterial cell.

If the cell-free lysate to be used is derived from a cell or microorganism which is different to the natural host of the virus or bacteriophage, at least one bacteriophage-host specific expression factor and/or a nucleic acid encoding the at least one bacteriophage-host specific expression factor, preferably a transcription factor, can be added, if necessary.

According to one embodiment of the invention, at least one nucleotide sequence encoding the at least one bacteriophage-host specific expression factor or any other protein preferably selected from the group comprising co-factors, chaperons, polymerases, transcription regulatory factors is cloned into the cell strain used to provide the cell lysate. The nucleotide sequence is expressed within the cell before lysis. Thus, after lysis the cell lysate already contains all nucleic acids and/or proteins necessary for the amplification of the virus or bacteriophage, in particular at least one host specific expression factor or any other protein selected from the group comprising co-factors, chaperons, polymerases, transcription regulatory factors and/or any mixture thereof.

Further modifications of the synthetic bacteriophage to be provided can be achieved by adding a modified protein to the cell-free expression system and/or by adding a nucleic acid encoding that modified protein. This protein itself can be of natural amino acid sequence or artificially modified for optimization.

The cell-free synthesis of proteins, i.e. outside living cells, has several advantages over cellular expression. This is all the more true if proteins are expressed that are toxic for the bacteria or non-natural amino acids shall be inserted into the proteins.

The production of bacteriophages in a cell-free lysate may also by carried out without any bacteriophage host bacteria, which makes it possible to produce them locally, e.g. in a laboratory without biosafety-level. At the same time the amount of toxic by-products that are produced by a method according to the present invention is reduced, since lysed pathogenic host bacteria are not present.

Another advantage is that if the *in vitro* method is based on a cell-free lysate only the genome of the added virus or bacteriophage is applied and thus, a pure solution of viruses or bacteriophage can be produced, without any pro-viruses or pro-phages. The cell-free expression in combination with the *in vitro* reproduction of nucleic acids and in particular the genome represents a broadly applicable method that accelerates the application of bacteriophages in research and development and biomedical applications.

During assembly step (d) the expressed proteins preferably self assemble to the inventive multi-peptide structure, in particular synthetic bacteriophage. Self-assembly can be supported and/or initiated by the addition of supporting factors, e.g. PEG, certain buffer systems e.g. standard phosphate buffered saline und in particular ions. Exemplary ions include, but are not limited to, $Li^+$, $Na^+$, $K^+$ and $Cs^+$ as well as divalent ions such as $Ca^{2+}$ or $Mg^{2+}$. In certain embodiments, the concentration of the monovalent and/or divalent ions is at least 5 mM, 10 mM, 20 mM or 50 mM in order to induce or substantially accelerate self-assembly. Self-assembled multi-peptide structures can be provided with high reproducibility and uniformity.

The inventive method enables the provision of a composition comprising different synthetic bacteriophages having a statistical combination of the functional sites described herein, such as different tail fibre assemblies. The combination of, for example, homologous tail fibre proteins during the assembly of the expressed proteins during step (d) at a functional site as described herein entails a combinatorial expansion of the possible binding sites of the provided composition comprising different synthetic bacteriophages due to the possibility of the different structure of the tail fibres. According to the invention, this combinatorial expansion is achieved in a simple manner by self-assembly at the protein level.

The concentration of multi-peptide structures, in particular bacteriophages, generated *in vitro* may be so high that the corresponding composition and/or formulation can directly be applied in a therapy with phages without a further concentration step. If needed, a further concentration step, in particular after purification, is of course also within the scope of the present invention.

A further aspect of the invention relates to a multi-peptide structure, in particular a synthetic bacteriophage, provided by the methods described herein. All embodiments described herein relate preferably to self-assembled multi-peptide structures, in particular synthetic bacteriophages.

Another aspect of the invention relates to a composition comprising the multi-peptide structure, in particular synthetic bacteriophage, as described herein.

A further aspect of the invention relates to a composition comprising two or more types of multi-peptide structures, in particular synthetic bacteriophages, as described herein, wherein the two or more types of the multi-peptide structure, in particular synthetic bacteriophage, have homologous tail fibre and/or capsid proteins or have different mutations in the tail fibre and/or capsid proteins. Such a composition comprises different synthetic bacteriophages which may have a statistical combination of the functional sites set up by the at least two homologous peptide, such as a statistical combination of tail fibres with regard to their protein composition as well as their positioning in the multi-peptide structure.

It is preferred that the two or more types of the multi-peptide structure, in particular synthetic bacteriophage, have different host ranges.

The inventive compositions may comprise carriers and/or vehicles as described below. According to preferred embodiments, the compositions are pharmaceutical compositions.

A further aspect of the invention relates to the use of multi-peptide structure, in particular synthetic bacteriophage, provided as described herein for use in medicine, chemistry, biotechnology, agriculture and/or food industry. Such uses are not limited to bacteria related fields and comprise, for example, the use as vaccine, for example against infections as well as tumors, and the use as a delivery vehicle for any kind of drug, in particular anti-cancer drugs, to a target cell, in particular a human target cell.

The use in bacteria related fields is, however, preferred. Multi-peptide structures, in particular synthetic bacteriophages, may in particular be used as bacterial population control alternatives to antibiotics. They are much more specific than antibiotics and are typically harmless not only to the host organism but also to other beneficial bacteria, such as the gut microbiota, as they are very selective in the strains of bacteria, they are effective against. Thereby the chances of opportunistic infections are significantly reduced. Advantages include further reduced side-effects and reduced risk of the bacterium's developing resistance. Because phages replicate in vivo, in certain embodiments small effective doses can be used. These unique properties make them highly promising antimicrobials.

Accordingly, the multi-peptide structures, in particular synthetic bacteriophages, provided herein may be part of a pharmaceutical composition. Such a composition may comprise (i) at least one bacteriophage strain capable of producing a lytic infection and (ii) a pharmaceutically acceptable carrier and/or vehicle. Of course, such composition may comprise synthetic bacteriophage mixtures.

The pharmaceutical compositions may be used in combination with antibiotics for the purpose of treating bacterial infections and/or to treat antibiotic resistant bacteria. In particular, bacteriophages tend to be more successful than antibiotics where there is a biofilm covered by a polysaccharide layer, which antibiotics typically cannot penetrate. According to a preferred embodiment, the composition may be freeze dried.

"Pharmaceutically acceptable carrier" relates to pharmaceutically-acceptable, fillers or diluents used to formulate pharmaceutical compositions for animal or human administration. The pharmaceutical compositions may further comprise pharmaceutically acceptable auxiliary agents, and optionally other therapeutic agents. In particular for oral administration it is preferred to add an antacid, i.e. a substance which neutralizes stomach acidity, thereby increasing the number of phages surviving passage through the stomach.

"Vehicle" as used herein refers to any compound or combination known to the person skilled in the art known to be useful in formulating a composition, in particular a pharmaceutical composition. Such a vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material.

In tablets, the active agent (i.e. multi-peptide structure, in particular bacteriophage) may be mixed with a vehicle having the necessary compression properties in suitable proportions and compacted in the shape and size desired.

Suitable solid vehicles include, for example calcium phosphate, magnesium stearate, talc, sugars, lactose, dextrin, starch, gelatin, cellulose, polyvinylpyrrolidine, low melting waxes and ion exchange resins.

In another embodiment, the pharmaceutical vehicle may be a gel and the composition may be in the form of a cream or the like.

The dose and regimen of administration of a pharmaceutical composition will necessarily be dependent upon the therapeutic effect to be achieved (e.g. treatment of IBD) and may vary with the particular bacteriophage strains in the composition, the route of administration, and the age and condition of the individual subject to whom the pharmaceutical composition is to be administered.

If the pharmaceutical composition is to be administered with one or more antibiotics, it may be simultaneously, separately or sequentially administered.

Pharmaceutical compositions and routes of administration include those suitable for or via oral (including buccal, sublingual and intraorbital), rectal, nasal, topical (including transdermal), ocular, vaginal, bronchial, pulmonary or parenteral (including subcutaneous, intramuscular, intravenous, intradermal, intraperitoneal, intrapleural, intravesicular and intrathecal) administration or administration via an implant. In particular a freeze-dried composition may be administered orally, preferably on form of a pill.

Agriculture comprises plant agriculture as well as animal agriculture, wherein bacteriophages can replace or complement antibiotics and/or pesticides.

Bacterial pathogens are associated with a couple of plant diseases which can also be treated with synthetic bacteriophages provided herein. Non limiting examples for bacterial plant diseases which might be treated are leaf blight on onion, potato scab or soft rot of potato, bacterial wilt on tobacco, citrus bacterial spot or citrus canker of citrus, fire blight on apple, black rot of cabbage, soft rot of calla lilies, bacterial wilt of tomato and bacterial spot of peach.

The use of the inventive multi-peptide structures, in particular synthetic bacteriophages, in animal agriculture is exemplified by the use against Salmonella, pathogenic E. *coli,* Clostridium, and Campylobacter for the poultry industry and against Salmonella and pathogenic E. *coli for* the pig industry.

Multi-peptide structures, in particular synthetic bacteriophages, provided herein may also be used to safen food products, and to forestall spoilage bacteria. Since 2006, the United States Food and Drug Administration (FDA) and United States Department of Agriculture (USDA) have approved several bacteriophage products. For example, LMP-102 (Intralytix) was approved for treating ready-to-eat (RTE) poultry and meat products. In that same year, the FDA approved LISTEX using bacteriophages on cheese to kill Listeria monocytogenes bacteria.

With regard to biotechnology related uses, the methods described herein as well as multi-peptide structures, in particular synthetic bacteriophages, provided herein can, for example, be used for phage display related application.

The invention further includes a kit comprising a pharmaceutical composition of the invention and instructions for the use of the composition for a use as hereinbefore described, optionally together with packaging material.

Further aspects relate to the use of multi-peptide structures, in particular synthetic bacteriophages, provided herein in methods for the prevention and or treatment of a bacterial infection as well as for avoiding bacterial growth.

The present invention is in particular described by the following items:

1. A multi-peptide structure comprising at least one heterogenous functional site wherein the at least one heterogenous functional site being composed of at least two homologous peptides, which differ by at least one amino acid.

2. The multi-peptide structure according to item 1, wherein the at least two homologous peptides are derived from different bacteriophages and/or are engineered in a different way.

3. The multi-peptide structure according to item 1 or 2, comprising a bacteriophage derived nucleic acid, in particular bacteriophage genome, encoding at least one homologous peptide less than comprised by the multi-peptide structure.

4. The multi-peptide structure of any of the preceding items, wherein the functional site is selected from head (capsid), tail, spike, sheath, tube, baseplate or fiber component of a bacteriophage, preferably a capsid or fiber component and even more preferably a fibre tail component.

5. The multi-peptide structure of any of the preceding items, wherein the at least two homologous peptides are selected from the group comprising receptor binding proteins, tail fibre proteins or tail fibre loops, and/or capsid proteins.

6. The multi-peptide structure of any of the preceding items, wherein the multi-peptide structure comprises at least two homologous bacteriophage tail fibre proteins and/or at least two homologous bacteriophage capsid proteins, preferably derived from different bacteriophages.

7. The multi-peptide structure of any of the preceding items,
wherein at least one of the at least two homologous bacteriophage peptides is engineered.

8. The multi-peptide structure of any of the preceding items,
wherein the multi-peptide structure is capable of binding on the surface of a cell, in particular a bacterial cell.

9. The multi-peptide structure of any of the preceding items,
wherein the homologous tail fibre proteins are specific for different hosts.

10. The multi-peptide structure of any of the preceding items,
wherein the homologous tail fibre proteins are specific for the same host.

11. The multi-peptide structure of any of the preceding items,
wherein the homologous tail fibre proteins are specific for gram positive and/or gram negative bacteria, in particular gram positive and gram negative bacteria.

12. The multi-peptide structure of any of the preceding items,
wherein the multi-peptide structure is assembled, preferably self-assembled, and/or resembles a headless phage.

11. The multi-peptide structure of any of the preceding items,
wherein the different bacteriophages are selected from different wild-type bacteriophages.

13. The multi-peptide structure of any of the preceding items,
wherein the different bacteriophages, in particular wild-type bacteriophages, are selected from the group comprising Caudovirales (familiy of Myoviridae, Autographiviridae, Podoviridae, Ackermannviridae, and Siphoviridae), Leviviridae, Tectiviridae, Corticoviridae, Plasmaviridae, Sphaerolipoviridae, Inoviridae, Microviridae, Picobirnaviridae and/or Cystoviridae.

15. The multi-peptide structure of any of the preceding items,
wherein at least one of the receptor binding proteins and/or capsid forming proteins comprises non-naturally generated mutations, e.g. resulting from deletions, insertions and/or substitutions within the corresponding encoding nucleic acid.

16. The multi-peptide structure of any of the preceding items,
wherein the multi-peptide structure is labelled.

17. Method, in particular *in vitro* method, for providing multi-peptide structures, in particular synthetic bacteriophages, comprising the steps of

(a) providing an expression system, in particular a cell-free expression system,
(b) adding nucleic acids encoding homologous bacteriophage proteins, preferably derived from different bacteriophages, in particular homologous bacteriophage tail fibre proteins and/or homologous bacteriophage capsid proteins, to the expression system,
(c) expressing the nucleic acids encoding the homologous proteins, in particular homologous phage tail fibre proteins and/or homologous phage capsid proteins,
(d) assembling of the expressed homologous proteins, in particular homologous phage tail fibre proteins and/or homologous phage capsid proteins, to provide the assembled multi-peptide structures, in particular the synthetic bacteriophages, and optional
(e) isolating of the assembled multi-peptide structures, in particular the synthetic bacteriophages.

18. The method of item 17,
wherein the expression system is a cell-free expression system.

19. The method of any of items 17 or 18,
wherein the cell-free expression system is host independent and preferably selected from cell lysates or artificial expression systems.

20. The method of any of items 17-19,
wherein the cell lysates are derived from microorganisms, in particular bacteria, yeast, insects, mammals, plants and/or are artificial.

21. The method of any of items 17-20,
comprising the addition of bacteriophage-host specific factors.

22. The method of any of items 17-21,
wherein in step (b) the nucleic acids encoding homologous proteins, preferably of at least two different bacteriophages, are added in a predetermined ratio to determine the composition of the assembled multi-peptide structure, in particular synthetic bacteriophage, to be provided.

23. The method of any of items 17-22,
wherein the nucleic acids encoding proteins of the at least two different bacteriophages are DNA and/or RNA molecules, such as PCR products, plasmid vectors, native DNA and/or RNA molecules, chemically or biologically synthesized molecules, artificial chromosomes, in particular yeast artificial chromosomes and/or bacterial artificial chromosomes, etc.

24. The method of any of items 17-23, wherein the DNA and/or RNA molecules are essentially complete genomes of bacteriophages.

25. The method of any of items 17-24, wherein the at least two different DNA and/or RNA molecules are one essentially complete genome of a bacteriophage, and at least one nucleic acid molecule encoding at least one selected homologous protein, in particular a tail fibre or capsid protein, derived from another bacteriophage.

26. The method of any of items 17-25, wherein at least one of the homologous protein encoding nucleic acids is modified, in particular modified by non-naturally mutations such as deletion, insertion and/ or substitution.

27. The method of any of items 17-26, wherein at least one homologous protein derived from another bacteriophage, is a capsid protein and/or tail fibre protein.

28. The method of any of items 17-27, wherein only one type of nucleic acid molecule, in particular an expression vector, encoding homologous proteins, preferably of at least two different bacteriophages, is added.

29. Multi-peptide structure, in particular a synthetic bacteriophage, provided by the method of any of items 17-29.

30. Composition comprising the multi-peptide structure, in particular synthetic bacteriophage, according to any of items 1-16 or 29.

31. Composition comprising two or more types of multi-peptide structure, in particular synthetic bacteriophages, according to any of items 1-16 or 29, wherein the two or more types of the multi-peptide structure, in particular synthetic bacteriophages, have homologous tail fibre and/or capsid proteins or have different mutations in the tail fibre and/or capsid proteins.

32. The composition of item 31, wherein the two or more types of the multi-peptide structure, in particular synthetic bacteriophage, have different host ranges.

33. An universal anti-microbial agent comprising the multi-peptide structure of any of items 1-16 or 29 or the composition according to any of items 28-30.

34. Multi-peptide structure, in particular a synthetic bacteriophage, according to any of items 1-16 or 29 or the composition according to any of items 28-30 for use in medicine, chemistry, biotechnology, agriculture and/or food industry.

35. The multi-peptide structure, in particular a synthetic bacteriophage, or composition according to item 34 for use in a method for the prevention and or treatment of a bacterial infection.

36. Use of a multi-peptide structure, in particular a synthetic bacteriophage, according to any of items 1-16 or 29 or composition according to any of items 30-32 for avoiding bacterial growth.

**Figures**

Figure 1:    General schematic Figure of a T7 like bacteriophage with a icosahedral head, a tail and tail-fiber

Figure 2:    Illustration of the triangular lattice built from asymmetric building blocks to form an icosahedron-like structure. The possible contact points of the structure are marked with the letters A-E.

Figure 3:    Concentration (plaque forming units/ml (PFU/ml)) of different simultaneously expressed particles in cell extract plated on different hosts.

**Example**

The process demonstrated below provides a simultaneous all *in vitro* method for the production of multi-peptide structure in a cell-free system.

For this purpose, several coding strands of genetic information (DNA/RNA) were simultaneously added to a cell-free reaction and incubated. This genetic information contains the appropriate structural information, such as the structural information of the asymmetric building blocks from which, for example, a capsid is formed. These are expressed simultaneously in this system, and the specificity of the structure of the proteins leads to a subsequent self-assembly. By varying the concentration of the added coding genetic information, it is also possible to adjust the final concentration of the resulting multi-peptide structure for further use.

The process demonstrated here provides a method for the production of a multi-peptide structure as described herein in a cell-free system.

For this purpose, several coding strands of genetic information (DNA/RNA) are simultaneously added to a cell-free reaction and incubated. This genetic information must contain the appropriate structural information, such as the structural information of the asymmetric building blocks from which, for example, a capsid is formed.

The genetic information is expressed simultaneously in the system. The specificity of the protein structure leads to a subsequent assembly.

By varying the concentration of the added coding genetic information, it is also possible to adjust the final concentration of the resulting multi-peptide structure for further use.

The first step involves the preparation of an *E.coli* cell lysate. Here, an *E.coli* based cell extract is used, as these have the highest expression capacity so far (Caschera et al. 2013) and its composition is well known.

The second step involves the provision of the genetic information (DNA/RNA), which must encode specific structural elements. This genetic information must be isolated or physically available e.g. in the form of PCR product, native DNA/RNA, chemically synthesized, as a plasmid or as a yeast artificial chromosome (list not exhaustive).

As an exemplifying embodiment, a genome from the species of teseptimaviruses with a T=7 symmetry is used (DSM 4623) and a similar genome from the same species (DSM No 4621), which are produced simultaneously in a single cell-free reaction. Teseptimavirus is a synonym for T7 phage group or T7-like virus.

The first step for bacteriophage assembly involved the preparation of an E. *coli* Rosetta™(DE3) cell lysate.

For this, a cell culture after reaching a certain optical density was harvested and washed. Subsequently, the bacteria were lysed by adding lysozyme and further treated under ultrasound. The latter step improves lysis. The cell membrane, DNA and small metabolites were removed. The remaining solution contained the cell-free extract, which is capable of performing the expression. A buffer containing small metabolites supplementing expression was added.

The genomes of the two teseptima viruses were then extracted with the aid of a genome extraction kit (Zymo Research).

These two prepared DNA strands were added to the cell-free system. In this system, the expression of the genes of one teseptimavirus and of the other took place simultaneously. After incorporating the DNA into the capsid, the expressed proteins assembled into fully functional teseptimaviruses, i.e. functional particles.

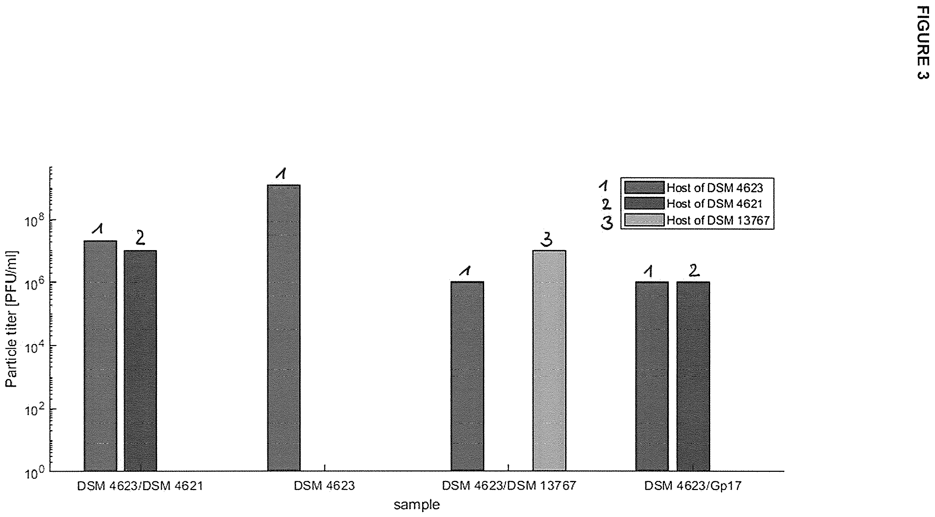

The presence of the functional particles was detected by a spotting assay on the respective host and the concentration determined. The expression of the proteins can also be optionally detected by mass spectrometry and the identity of the genetic information by sequencing (c.f. Figure 3).

Figure 3 shows the concentration (plaque forming units/ml (PFU/ml)) of different simultaneously expressed particles in cell extract plated on different hosts.

Blue represents plating on host DSM 613 which is the host for DSM 4623, red represents plating on host EcoR16 which is the host for DSM 4621, and yellow represents plating on host DSM 5695 which is the host for DSM 13767.

In sample one, the genome of DSM 4623 and DSM 4621 were simultaneously added to the cell extract and tested against the respective hosts, in the second sample only the genome of DSM 4623 was added to the cell extract and tested against the respective host, and in sample 3 the genome of DSM 4623 and DSM 13767 were added to the cell extract and tested against the respective host. In the last sample the genome of DSM 4623 and a plasmid encoding the gene Gp17 of DSM 4621 were added to the cell extract and tested against the respective hosts.

When the genome of DSM 4621 and DSM 4623 were present in the cell extract at the same time, plaques could be detected both when plating with the host for DSM 4623 and the host for DSM 4621 (Figure 2).

A similar result was obtained when only part of the genome of DSM 4621 was added (plasmid encoding Gp17) and at the same time the whole genome of DSM 4621.

The genome of an emesvirus with a triangulation number of T=3 and the genome of DSM 4623 were also added together in the cell extract. Here, it was also demonstrated that more than $10^6$PFU/ml particles were present against both the host of DSM 4623 and the host of DSM 13767.

This demonstrated the simultaneous production of particles, i.e. multi-peptide structures for different hosts for gene transfer with different as well as the same triangulation numbers T in a cell-free system.

**Claims**

1.  A multi-peptide structure comprising at least one heterogenous functional site wherein the at least one heterogenous functional site is composed of at least two homologous peptides, which differ by at least one amino acid.

2.  The multi-peptide structure according to claim 1, wherein the at least two homologous peptides are derived from different bacteriophages and/or are engineered in a different way.

3.  The multi-peptide structure according to claim 1 or 2, comprising a bacteriophage derived nucleic acid, in particular bacteriophage genome, encoding at least one homologous peptide less than comprised by the multi-peptide structure.

4. The multi-peptide structure of any of the preceding claims, wherein the functional site is selected from head (capsid), tail, spike, sheath, tube, baseplate or fiber component of a bacteriophage, preferably a capsid or fiber component and even more preferably a fibre tail component.

5. The multi-peptide structure of any of the preceding claims, wherein the at least two homologous peptides are selected from the group comprising receptor binding proteins, tail fibre proteins or tail fibre loops, and/or capsid proteins.

6. The multi-peptide structure of any of the preceding claims,
wherein the multi-peptide structure comprises at least two homologous bacteriophage tail fibre proteins and/or at least two homologous bacteriophage capsid proteins, preferably derived from different bacteriophages.

7. Method, in particular *in vitro* method, for providing multi-peptide structures, in particular synthetic bacteriophages, comprising the steps of

(a) providing an expression system, in particular a cell-free expression system,
(b) adding nucleic acids encoding homologous bacteriophage proteins, preferably derived from different bacteriophages, in particular homologous bacteriophage tail fibre proteins and/or homologous bacteriophage capsid proteins, to the expression system,
(c) expressing the nucleic acids encoding the homologous proteins, in particular homologous phage tail fibre proteins and/or homologous phage capsid proteins,
(d) assembling of the expressed homologous proteins, in particular homologous phage tail fibre proteins and/or homologous phage capsid proteins, to provide the assembled multi-peptide structures, in particular the synthetic bacteriophages, and optional
(e) isolating of the assembled multi-peptide structures, in particular the synthetic bacteriophages.

8. The method of claim 7,
wherein the expression system is a cell-free expression system, wherein the cell-free expression system is preferably host independent and preferably selected from cell lysates or artificial expression systems.

9. The method of any of claims 7 or 9,
comprising the addition of bacteriophage specific host factors.

10. The method of any of claims 7-9,
wherein at least one of the protein encoding nucleic acids is modified, in particular modified by non-natural mutations such as deletion, insertion and/ or substitution.

11. Composition comprising the multi-peptide structure, in particular synthetic bacteriophage, according to any of claims 1-6.

12. Composition comprising two or more types of multi-peptide structure, in particular synthetic bacteriophage, according to any of claims 1-6, wherein the two or more types of the multi-peptide structure, in particular synthetic bacteriophage, have homologous tail fibre and/or capsid proteins or have different mutations in the tail fibre and/or capsid proteins.

13. An universal anti-microbial agent comprising the multi-peptide structure of any of claims claim 1-6 or the composition according to any of claims 11 and 12.

14. Multi-peptide structure, in particular a synthetic bacteriophage, according to any of claims 1-6 or the composition according to any of claims 11-13 for use in medicine, chemistry, biotechnology, agriculture and/or food industry.

15. Use of a multi-peptide structure, in particular a synthetic bacteriophage, according to any of claims 1-6 or composition according to any of claims 11-13 for avoiding bacterial growth.

**FIGURE 1**

**FIGURE 2**

FIGURE 3

Europäisches
Patentamt

European
Patent Office

Office européen
des brevets

# EUROPEAN SEARCH REPORT

Application Number

EP 22 19 6655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DUNNE MATTHEW ET AL: "Reprogramming Bacteriophage Host Range through Structure-Guided Design of Chimeric Receptor Binding Proteins", CELL REPORTS, vol. 29, no. 5, 1 October 2019 (2019-10-01), pages 1336-1350.e4, XP093031187, US ISSN: 2211-1247, DOI: 10.1016/j.celrep.2019.09.062 * abstract * * figure 3 * * page 1341, 2nd column; page 1346, 2. column, lines 8-10 * | 1-5,7, 9-15 | INV. A61P31/04 C12N7/00 C12P21/00 |
| X | EMSLANDER QUIRIN ET AL: "Cell-free production of personalized therapeutic phages targeting multidrug-resistant bacteria", CELL CHEMICAL BIOLOGY, ELSEVIER, AMSTERDAM, NL, vol. 29, no. 9, 11 July 2022 (2022-07-11), page 1434, XP087181024, ISSN: 2451-9456, DOI: 10.1016/J.CHEMBIOL.2022.06.003 [retrieved on 2022-07-11] * figure 4 * * abstract * * page 1440, column 2, paragraph 2 * | 1-15 | TECHNICAL FIELDS SEARCHED (IPC) A61P C07K C12N C12P |

-/--

The present search report has been drawn up for all claims

| Place of search | Date of completion of the search | Examiner |
|---|---|---|
| Munich | 14 March 2023 | Voigt-Ritzer, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 19 6655

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARZARI R ET AL: "Extending filamentous phage host range by the grafting of a heterologous receptor binding domain", GENE, ELSEVIER AMSTERDAM, NL, vol. 185, no. 1, 31 January 1997 (1997-01-31), pages 27-33, XP004093150, ISSN: 0378-1119, DOI: 10.1016/S0378-1119(96)00623-3 * the whole document * * page 28, column 1, paragraph 3 * * figure 1 * | 1-15 | |
| A | SMITH MARK T. ET AL: "The incorporation of the A2 protein to produce novel Q[beta] virus-like particles using cell-free protein synthesis", BIOTECHNOLOGY PROGRESS, vol. 28, no. 2, 28 November 2011 (2011-11-28), pages 549-555, XP093030897, ISSN: 8756-7938, DOI: 10.1002/btpr.744 * page 550, column 1, paragraph 2 * | 1-15 | |
| A | WO 2020/128108 A1 (ELIGO BIOSCIENCE [FR]) 25 June 2020 (2020-06-25) * paragraph [0009] * * figure 9 * * examples 2,3 * | 1-15 | TECHNICAL FIELDS SEARCHED (IPC) |

The present search report has been drawn up for all claims

| Place of search | Date of completion of the search | Examiner |
|---|---|---|
| Munich | 14 March 2023 | Voigt-Ritzer, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 6655

This annex lists the patent family members relating to the patent documents cited in the above-mentioned European search report.
The members are as contained in the European Patent Office EDP file on
The European Patent Office is in no way liable for these particulars which are merely given for the purpose of information.

14-03-2023

| Patent document cited in search report | | Publication date | Patent family member(s) | | Publication date |
|---|---|---|---|---|---|
| WO 2020128108 | A1 | 25-06-2020 | CA | 3120160 A1 | 25-06-2020 |
| | | | CN | 113498435 A | 12-10-2021 |
| | | | EP | 3898953 A1 | 27-10-2021 |
| | | | IL | 283796 A | 29-07-2021 |
| | | | JP | 2022514700 A | 14-02-2022 |
| | | | KR | 20210107053 A | 31-08-2021 |
| | | | WO | 2020128108 A1 | 25-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FALGENHAUER ; S. VON SCHÖNBERG ; C. MENG ; A. MUCKL ; K. VOGELE ; Q. EMSLANDER ; C. LUDWIG ; F. C. SIMMEL.** *Chem-BioChem,* 2021, vol. 22, 2805